# EUROPEAN PATENT APPLICATION

(11) **EP 4 276 843 A1**
(43) Date of publication of application: **15.11.2023**
(21) Application number: 22305692.0
(22) Date of filing: 10.05.2022
(51) Int. Cl.: G16H 20/30, G16H 50/70

(54) **METHOD AND SYSTEM FOR AUTOMATICALLY PROVIDING ADAPTED ELECTRONIC TRAINING PLANS TO INDIVIDUALS OF A TARGETED GROUP OF INDIVIDUALS**

(71) Applicant: BULL SAS, 78340 Les Clayes-Sous-Bois (FR)
(72) Inventor: PATANKAR, Dheeraj, 411038 Pune (IN)
(74) Representative: Argyma

(57) **Abstract**

A method for automatically providing an electronic training plan to each individual of a targeted group of individuals called "irregulars" (IRR), said targeted group comprising individuals who do not perform at least one activity on a periodic basis, the targeted group being a part of a larger group called set of individuals (SIND), said set of individuals comprising another group called "regulars" (REG), disjointed from the group of irregulars (IRR) and comprising individuals who do perform at least one activity on a periodic basis, each individual of the set of individuals (SIND) being characterized by a set of physiological data and a set of medical data.

## Description

### [Field of the invention]

The field of the invention relates to health and safety and, more particularly, to a method and device for automatically providing adapted electronic training plans to individuals of a targeted group of individuals.

### [Background of the invention]

In today's world, lifestyle related problems severely cause productivity losses across multiple countries around the world. The estimated cost of such losses is in billions of euros for many countries (multiple references are available publicly). Health insurance claims and premiums have an increasing trend year over year, more so in post Covid situation.

Many individuals are advised to remain physically active to manage stressful work lifestyles, reduce stress, strict diet control and increase immunity to sustain an acceptable productivity level. Typically, they take up endurance sports like cycling, running in mid-life age, often end up stretching limits, trying to mimic someone's training plan, going through online material or false advice etc.

Since the adaptation of an endurance activity is subjective to an individual's physiology and anatomy, many of them face a risk of getting injured. The direct/ indirect implication of such injuries is that an individual gets demotivated, causing further productivity loss.

However, if monitored, controlled and guided accurately, research has shown that physical activities pay off well in the long run thereby improving the productivity of an individual year over year. In this regard, insurance service providers may for example offer add-on service to monitor, manage and guide to improve productivity to individuals, improve one's overall health and get customer commitment in return.

In one existing solution, people are advised to follow pre-determined training plans. However, these training plans are not necessarily adapted to each individual and may therefore prove not to be efficient.

In another existing solution, people are advised to follow tailor-made training plans conceived by some medical staff. Therefore, in this solution, a tailor-made training plan must be created for each individual depending on their physiological features, which can be long and costly.

It is therefore an object of the present invention to provide a solution to remedy at least in part to these drawbacks.

### [Summary of the invention]

To this end, the present invention concerns a method for automatically providing an electronic training plan to each individual of a targeted group of individuals called "irregulars", said targeted group comprising individuals who do not perform at least one activity on a periodic basis, the targeted group being a part of a larger group called set of individuals, said set of individuals comprising another group called "regulars", disjointed from the group of irregulars and comprising individuals who do perform at least one activity on a periodic basis, each individual of the set of individuals being characterized by a set of medical data and a set of physiological data, said set of physiological data comprising basic physiological data and dynamic physiological data, said basic physiological data and said medical data being stored in a database, said method comprising the steps, operated by a computer system, of:
- measuring dynamic physiological data of each individual of the set of individuals,
- sending said measured dynamic physiological data to said database via a communication network,
- associating, for each individual, their dynamic physiological data with their basic physiological data and their medical data,
- collecting a data subset of the set of physiological data and a data subset of the set of medical data for each individual of the set of individuals, said medical data subset comprising preferably at least the age and the weight of each individual,
- for each individual of the group of regulars, determining in the collected physiological data subset the data which are correlated with peak performances and deriving parameters associated with the determined peak performances data,
- determining a plurality of subsets of the set of individuals, called "classes", based on the medical data subset and/or the basic physiological data of each individual in order to group individuals at least by age and/or weight categories,
- generating, for each class, a common electronic training plan based on the derived parameters of the regulars of said class,
- sending to each class, via the communication network, said electronic training plan to the irregulars of said class.

Regulars are people who have a regular activity, preferably at least one activity every week, preferably for at least one hour, preferably for at least four weeks in a row. Irregulars are people who have an irregular activity, i.e. who do not have a regular activity. Preferably, irregular activity is characterized by a frequency of less than once a week over several weeks in a row, preferably at least four weeks in a row.

Medical data comprises at least the age of the individual. Preferably, the set of medical data also comprises the weight, the height, the gender and/or other medical-related data such as e.g. medical conditions like blood pressure, diabetes, hypertension, weekday and weekend work hours, work role, frequent health check-ups or doctor visits, prior procedures (any surgeries), unwell leaves, unscheduled leaves due to frequent illness, chronic conditions developed over the years, emergency hospital visits, unexpected shoot up in blood pressure or sugar levels.

Basic physiological data give an indication of the level of activity of the individual and may comprise an estimation of the activity habits of the individual like e.g. how many times a week the individual performs a sport activity, how many steps the individual walk each day, or any type of activity habit that may help to estimate whether the individual perform activities on a regular basis or not.

Dynamic physiological data are physiological data that are measured using sensors during an activity like a number of steps, a speed, a climbing height, etc.

A subset of data can be the whole set of data.

A parameter associated to a data correspond to the type of data.

The method according to the invention allows to generate and provide a training plan to people needing more activity based on data measured for people who do regularly have an activity. The method allows to provide a tailor-made training plan to irregulars based on data associated to regulars. The method may be used within the framework of health insurance to provide adapted rates and policies to customer based on their improvements using the electronic training plan.

Preferably, a periodic basis is at least once a week.

Preferably, the electronic training plan comprises the type and the frequency of activity/activities to perform.

According to an aspect of the present invention, each individual of the set of individuals being associated with a set of basic physiological data, the method comprises a preliminary step of separating the individuals of the set of individuals using the sets of basic physiological data of said individuals to define the group of regulars and the group of irregulars.

In an embodiment, the method comprises a step of collecting a set of basic physiological data e.g., using a survey or questionnaire, preferentially periodically, for example every year.

In an embodiment, the method comprises a step of collecting a set of medical data, e.g., using a survey or questionnaire, preferentially periodically.

In an embodiment, the method comprises, subsequently to the generation of a common electronic training plan for the irregulars of a class, a step of tuning said common electronic training plan for each irregular of said class using the physiological data subset and/or the medical data subset of said irregular.

In an embodiment, the method comprises measuring dynamic physiological data of each individual of the set of individuals using at least one measurement module, preferably a plurality of measurement modules.

In an embodiment, the method comprises measuring dynamic physiological data of each individual of the set of individuals on a periodic basis to update said data regularly, for example at least once a week.

In an embodiment, the method comprises monitoring specifically dynamic physiological data of the irregulars and suggesting improved electronic training plan based on the monitored dynamic physiological data.

In an embodiment, the method is used for managing insurance contracts of a set of individuals.

The invention also relates to a computer program comprising instructions which, when the program is executed by a computer, cause the computer to carry out the steps of the method as described here before.

The invention also relates to a computing device, for example a computer or a smartphone, for automatically providing an electronic training plan to each individual of a targeted group of individuals called "irregulars", said targeted group comprising individuals who do not perform at least one activity on a periodic basis, the targeted group being a part of a larger group called set of individuals, said set of individuals comprising another group called "regulars", disjointed from the group of irregulars and comprising individuals who do perform at least one activity on a periodic basis, each individual of the set of individuals being characterized by a set of medical data and a set of physiological data, said set of physiological data comprising basic physiological data and dynamic physiological data, said device being configured to be operatively coupled to a database comprising for each individual said set of basic physiological data and said of medical data, and to:
- receive, via a communication network, a data subset of the set of physiological data and a data subset of the set of medical data for each individual of the set of individuals, said medical data subset comprising at least the age and the weight of each individual,
- for each individual of the group of regular, determine in the collected physiological data subset the data which are correlated with peak performances and deriving the parameters associated with the determined peak performances data,
- determine a plurality of subsets of the set of individuals, called "classes", based on the medical data subset and/or the basic physiological data of each individual in order to group individuals at least by age and/or weight categories,
- generate, for each class, a common electronic training plan based on the derived parameters of the regulars of said class,
- send to each class, via the communication network, said electronic training plan to the irregulars of said class.

In an embodiment, each individual of the set of individuals being associated with a set of basic physiological data, the device is configured to retrieve at least basic physiological data for each individual of the set of individuals from the database and to separate the individuals of the set of individuals using said sets of basic physiological data of said individuals to define the group of regulars and the group of irregulars.

In an embodiment, the device is configured to collect a set of basic physiological data from each individual of the set of individuals and store said set in the database using a survey or a questionnaire, preferably on a periodical basis.

In an embodiment, the device is configured, subsequently to the generation of a common electronic training plan for the irregulars of a class, to tune said common electronic training plan for each irregular of said class using the physiological data subset and/or the medical data subset of said irregular.

In an embodiment, the device is configured to measure dynamic physiological data of each individual of the set of individuals using at least one measurement module, preferably a plurality of measurement modules.

In an embodiment, the device is configured to measure dynamic physiological data of each individual of the set of individuals on a periodic basis to update said data regularly, for example at least once a week.

In an embodiment, the device is configured to monitor specifically dynamic physiological data of the irregulars and to suggest improved electronic training plan based on the monitored dynamic physiological data.

In an embodiment, the device comprises the database.

The invention also relates to a database configured to receive and store medical data of each individual of the set of individuals, to receive and store physiological data of each individual of the set of individuals and to associate, for each individual, their physiological data with their medical data.

The invention also relates to a system for automatically providing an electronic training plan to each individual of a targeted group of individuals called "irregulars", said targeted group comprising individuals who do not perform at least one activity on a periodic basis, the targeted group being a part of a larger group called set of individuals, said set of individuals comprising another group called "regulars", disjointed from the group of irregulars and comprising individuals who do perform at least one activity on a periodic basis, each individual of the set of individuals being characterized by a set of physiological data and a set of medical data, said system comprising:
- a plurality of measurement modules,
- at least one database configured to receive and store medical data of each individual of the set of individuals, to receive and store physiological data of each individual of the set of individuals and to associate, for each individual, their physiological data with their medical data,
- at least one device, as described here before, operatively coupled to said at least one database via at least one communication network.

### [Brief description of the drawings]

These and other features, aspects, and advantages of the present invention are better understood with regards to the following Detailed Description of the Preferred Embodiments, appended Claims, and accompanying Figures, where:
[FIG. 1] Figure 1 schematically illustrates an embodiment of the system according to the invention.
[FIG. 2] Figure 2 schematically illustrates an example of functional implementation of the system of figure 1 for a health-insurance.
[FIG. 3] Figure 3 schematically illustrates an example of structural implementation of the system of figure 2.
[FIG. 4] Figure 4 schematically illustrates an embodiment of the method according to the invention.
[FIG. 5] Figure 5 schematically illustrates an example of functional implementation of the method of figure 4.

### [Detailed description]

The Specification, which includes the Summary of Invention, Brief Description of the Drawings and the Detailed Description of the Preferred Embodiments, and the appended Claims refer to particular features (including process or method steps) of the invention. Those of skill in the art understand that the invention includes all possible combinations and uses of particular features described in the Specification. Those of skill in the art understand that the invention is not limited to or by the description of embodiments given in the Specification. The inventive subject matter is not restricted except only in the spirit of the Specification and appended Claims. Those of skill in the art also understand that the terminology used for describing particular embodiments does not limit the scope or breadth of the invention. In interpreting the Specification and appended Claims, all terms should be interpreted in the broadest possible manner consistent with the context of each term. All technical and scientific terms used in the Specification and appended Claims have the same meaning as commonly understood by one of ordinary skill in the art to which this invention belongs unless defined otherwise. As used in the Specification and appended Claims, the singular forms "a", "an", and "the" include plural references unless the context clearly indicates otherwise. The verb "comprises" and its conjugated forms should be interpreted as referring to elements, components or steps in a non-exclusive manner. The referenced elements, components or steps may be present, utilized or combined with other elements, components or steps not expressly referenced. The verb "couple" and its conjugated forms means to complete any type of required junction, including electrical, mechanical or fluid, to form a singular object from two or more previously non-joined objects. If a first device couples to a second device, the connection can occur either directly or through a common connector. "Optionally" and its various forms means that the subsequently described event or circumstance may or may not occur. The description includes instances where the event or circumstance occurs and instances where it does not occur. "Operable" and its various forms means fit for its proper functioning and able to be used for its intended use. Where the Specification or the appended Claims provide a range of values, it is understood that the interval encompasses each intervening value between the upper limit and the lower limit as well as the upper limit and the lower limit. The invention encompasses and bounds smaller ranges of the interval subject to any specific exclusion provided. Where the Specification and appended Claims reference a method comprising two or more defined steps, the defined steps can be carried out in any order or simultaneously except where the context excludes that possibility.

Reference will now be made in detail to specific embodiments or features, examples of which are illustrated in the accompanying drawings. Wherever possible, corresponding or similar reference numbers will be used throughout the drawings to refer to the same or corresponding parts. Moreover, references to various elements described herein are made collectively or individually when there may be more than one element of the same type. However, such references are merely exemplary in nature. It may be noted that any reference to elements in the singular may also be construed to relate to the plural and vice-versa without limiting the scope of the disclosure to the exact number or type of such elements unless set forth explicitly in the appended claims.

Aspects of the present disclosure are directed to a computing resource, such as a server unit, implementing a connecting module capable of being disposed into a slot defined in the computing resource using minimum human effort. The connecting module includes levers capable of being pivotably deflected by a user to allow detachment of the connecting module from the computing resource. Likewise, the levers also enable the user to dispose the connecting module back into the slot of the computing resource. The connecting module eliminates use of any external tools to engage and disengage the connecting module from the computing resource.

### System 1

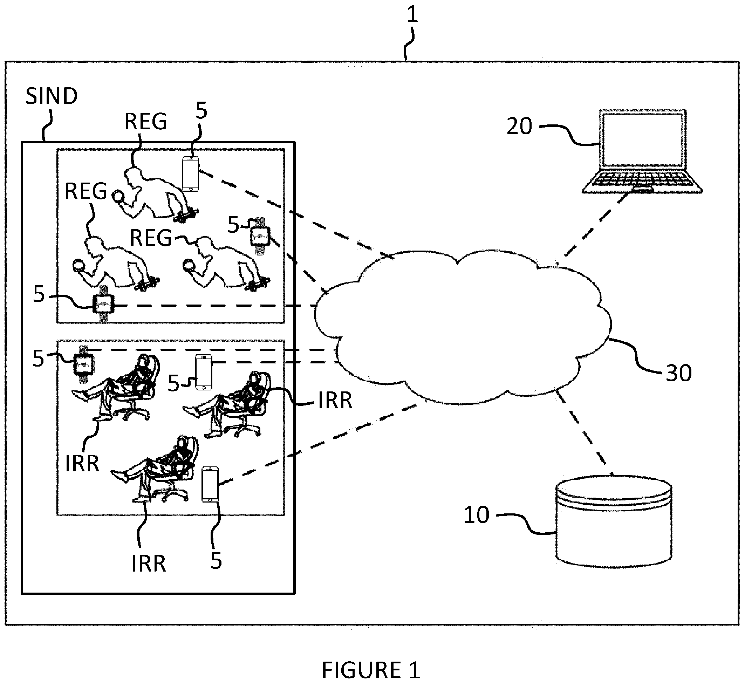

In reference to Figure 1, the system 1 comprises a plurality of measurement modules 5, a database 10 and a computing device 20.

The database 10 and the computing device 20 are operatively coupled, i.e. the database 10 and the computing device 20 are connected though a communication link or a communication network 30 in order for the computing device 20 to retrieve data from said database 10. In another embodiment, the computing device 20 could comprise the database 10. In other words, the database 10 might be embedded into the computing device 20.

The system 1 allows to automatically provide electronic training plans to individuals of a targeted group of individuals called "irregulars" IRR, said targeted group comprising individuals who do not perform at least one activity on a periodic basis.

The targeted group is a part of a larger group called set of individuals SIND, said set of individuals SIND comprising another group called "regulars" REG, disjointed from the group of irregulars IRR and comprising individuals who do perform at least one activity on a periodic basis.

Each individual of the set of individuals SIND is characterized by a set of medical data MD.

The set of medical data MD comprises at least the age of the individual. Preferably, the set of medical data MD also comprises the weight, the height, the gender and/or other medical-related data such as e.g. medical conditions like blood pressure, diabetes, hypertension, weekday and weekend work hours, work role, frequent health check-ups or doctor visits, prior procedures (any surgeries), unwell leaves, unscheduled leaves due to frequent illness, chronic conditions developed over the years, emergency hospital visits, unexpected shoot up in blood pressure or sugar levels.

Each individual of the set of individuals SIND is also characterized by a set of physiological data. Said set of physiological data comprises basic physiological data BPD and dynamic physiological data DPD.

Basic physiological data BPD give an indication of the level of activity of the individual and may comprise an estimation of the activity habits of the individual like e.g. how many times a week the individual performs a sport activity, how many steps the individual walk each day, or any type of activity habit that may help to estimate whether the individual perform activities on a regular basis or not. The basic physiological data BPD may be declared by the individual, e.g. via a questionnaire, or measured on the individual when said individual performs an activity. Dynamic physiological data DPD are physiological data that are measured using sensors during an activity like a number of steps, a speed, a climbing height, etc.

These lists of parameters of medical data MD and physiological data BPD/DPD are non-exhaustive and provided for illustrative purposes only.

Each individual of the set of individuals SIND is associated with at least one measurement module 5 such comprising at least one sensor that can measure dynamic physiological data DPD in real-time when said individual is performing an activity.

Such measurement module 5 may for example monitor heart rate, measure a time duration, a distance, a speed or any relevant data related to an activity. Such measurement module 5 may for example be a connected watch, a power sensor, a helmet, a heart frequency measuring belt, embedded sensors (on a bike, a gym machine, or else...), etc. This list of is non-exhaustive and provided for illustrative purposes only.

Each measurement module 5 is configured to send the measured dynamic physiological data DPD of the related individual to the database 10.

### Database 10

The database 10 is configured to receive medical data M D of each individual of the set of individuals via the communication network 30. The medical data MD may be entered into the database 10 though a user interface or via a file sent to the database 10. The database 10 is configured to store the received medical data MD of each individual of the set of individuals SIND.

The database 10 is configured to receive physiological data BPD/DPD of each individual of the set of individuals SIND via the communication network 30. The basic physiological data BPD may be entered into the database 10 though a user interface or via a file sent to the database 10. The dynamic physiological data DPD may be entered into the database 10 though a file sent to the database 10 by the measurement modules 5. The database 10 is configured to store the received physiological data BPD/DPD of each individual of the set of individuals SIND.

The database 10 is configured to, associate, for each individual, their physiological data BPD/DPD with their medical data MD.

### Computing device 20

The computing device 20 may be a personal computer, a server or any adapted computing device.

The computing device 20 is configured to retrieve from the database 10 a data subset of the set of physiological data BPD/DPD and a data subset of the set of medical data MD for each individual of the set of individuals SIND, said medical data MD subset comprising at least the age of each individual.

The computing device 20 is configured, for each individual of the group of regulars REG, to determine in the retrieved physiological data subset the data which are correlated with peak performances PP and deriving the parameters associated with the determined peak performances PP data.

The computing device 20 is configured to determine a plurality of subsets of the set of individuals SIND, called "classes", based on the medical data MD subset of each individual in order to group individuals at least by age and/or weight categories.

The computing device 20 is configured to generate, for each class, a common electronic training plan ETP based on the derived parameters of the regulars REG of said class.

The computing device 20 is configured to send to each class, via the communication network 30, said electronic training plan ETP to the irregulars IRR of said class.

### Communication network 30

The communication network 30 allows to exchange data between the measurement modules 5, the database 10 and the computing device 20. The communication network 30 may be based on or linked to or comprise a hyperscalar cloud platforms (e.g. AWS).

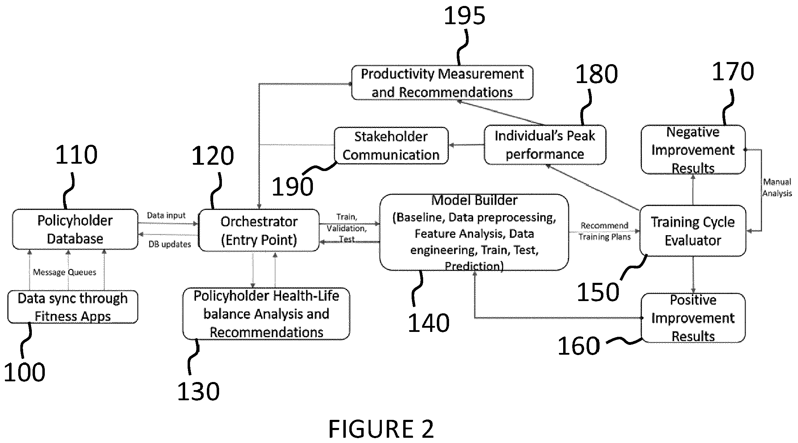

Figure 2 illustrates an example of generic functional architecture to implement the system of figure 1.

The individual policy holder data 100 are collected and synchronized through an application, e.g. a fitness app, that the individual may be using. The policyholder databased 110 is built using the data gathered through 100. The orchestrator 120 is the central program that manages overall data flow between various components of the system. Based on the baseline data collected, a policyholders health life balance analysis 130 can be performed to come up with recommendations. The model builder 140 create different ML models based on various steps such as data preprocessing, feature analysis, data engineering and standard ML methods and recommend training plans for individuals. The training cycle evaluator 150 assess an individual, as they progress through and at the end of the training cycle. The positive improvement results 160 are fed back into the ML model 140 for next training cycles. The negative improvement results 170 are processed through a manual analysis and feedback is provided to the training cycle evaluator. The individual's peak performances 180 are gathered based on the positive results. Based on the individual's peak performances, all the relevant stakeholders 190 are informed on the progress and achievements of that individual. Simultaneously the productivity measurement 195 can be quantified, over the training cycle period, such as reduction in frequent illness, better control over medical conditions such as blood pressure, hypertension, diabetes etc. Also, further recommendation can be provided for next training cycles.

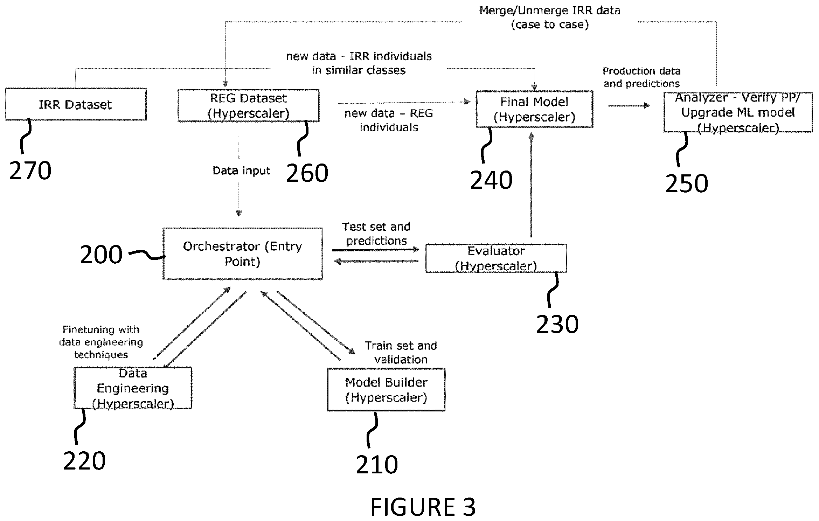

Figure 3 illustrates an example of detailed architecture to implement the system of figure 1. In this example, most of the components described use a hyperscalar platform (e.g. AWS) to store high volume data and perform high end calculations across hundreds of data parameters.

The orchestrator 200 is the central program that manages overall data flow to various core components of the Machine Learning (ML) system.

The model builder 210 creates the model using different hyper parameters and trains them using train dataset, a subset of REG dataset.

The data engineering 220 applies various data engineering techniques to the source data to finetune ML model and improve its prediction ability.

The evaluator 230 evaluates trained model using test data, predicts outcomes and generates performance baselines.

The final model module 240 is configured to select the final model after iterations of finetuning.

The analyzer 250 is used to verify the Peak performance PP of individuals and select/ unselect data for merging/unmerging into the original REG dataset.

The REG dataset 260 and IRR dataset 270 are the source datasets carrying information about respectively the REG and IRR individuals along with KPD, KMD, PP parameters.

### Example of operation

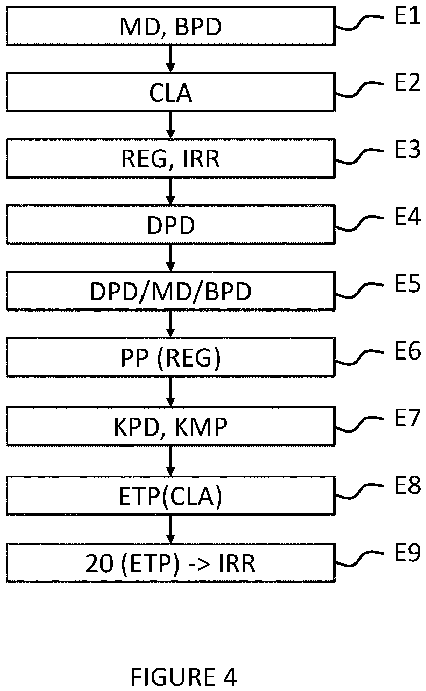

The method is described in reference to figure 4.

In a preliminary step E1, medical data MD and basic physiological data BPD of each individual of the set of individuals SIND are collected and stored in the database 10. For example, the data may be entered as answers to a questionnaire on a computer that sends said data to the database 10.

The computing device 20 determines in a step E2 a plurality of subsets of the set of individuals SIND, called "classes" CLA, based on the medical data MD subset of each individual in order to group individuals at least by age categories, and preferably also weight, height, gender, etc.

Once collected, medical data MD and basic physiological data BPD allow the computing device 20 in a step E3 to create two groups within the set of individuals: the regulars REG and the irregulars IRR.

Each time an individual of the set of individuals SIND performs an activity, like e.g. walking, running, swimming, ..., some dynamic physiological data DPD of said individual are measured using one or several measurement modules 5. The measured dynamic physiological data DPD may be collected on a device, like e.g. a smartphone, by a training application like e.g. GarminConnect^{®}, TrainingPeaks^{®} or Strava^{®}.

The measured dynamic physiological data DPD are collected for each individual of the set of individuals SIND and sent to the database 10 via the communication network 30 in a step E4.

Once received, the dynamic physiological data DPD of an individual are associated with the medical data MD and the basic physiological data BPD of said individual in a step E5.

For each individual of the group of regulars REG, in a step E6, the computing device 20 determines in the stored dynamic physiological data DPD set, the data which are correlated with peak performances PP and associates said data with the medical data MD of the individual. A peak performance PP could be goal specific to that individual (specific to running activity) like e.g., a time for a race, a threshold pace, a time spent in a heart rate zone, training volume over a month, long runs, etc.

Advantageously, the group of regulars REG may have data history of multiple years that would be the starting point to create the database for the set of individuals SIND, which may further be completed with subsequent activities from both the group of regulars REG and the group of irregulars IRR across all classes.

For each individual of the group of regulars REG, in a step E7, the computing device 20 derives the key physiological parameters KPD associated with the peak performances PP data determined for said individual and the key medical parameters KMP of said individual to establish a profile of the individual with regard to said individual's peak performances PP. Preferably, the data of each individual may be analyzed over a significant period of time, for example a few months.

Inherently, the individuals of the group of regulars REG have a significant history of data available comprising key physiological parameters KPD, key medical parameters KMP and peak performances PP. Machine learning techniques may be applied on this historical data to predict the next peak performances PP. For example, some peak performances PP may be known like e.g. 5K personal best time or a 21K distance constant pace, at a certain age, weight, medical history, nutrition plan, resting heart-rate, threshold pace, etc. Thus, next peak performances PP for that individual may be predicted with a new set of key physiological parameters KPD and key medical parameters KMP values. This prediction may be done by learning from the history of the individuals within the group of regulars REG with similar key physiological parameters KPD and key medical parameters KMP values. Artificial intelligence and/or machine learning may be applied to data available for the group of regulars REG to generate an electronic training plan ETP for the individuals of the group of irregulars IRR based on classes. At the same time, the same method may also be used to improve peak performances PP of the individuals of the group of regulars REG.

Classes CLA may be modified to group regular REG members having similar health and/or work lifestyle parameters like age, weight, working hours, ..., and how they adapt to stress changes using physical activities, keeping up with and achieving a peak performance PP to form a class profile.

The computing device 20 generates in a step E8, for each class CLA, a common electronic training plan ETP based on the derived parameters of the regulars REG of said class CLA.

The electronic training plan ETP is in priority intended for use by the group of irregulars IRR to improve their data and health. Preferably, the electronic training plan ETP is generated based on the model derived from the group of regulars REG having similar key physiological parameters KPD and key medical parameters KMP values (i.e. belonging to the same class CLA). The electronic training plan ETP is build in order to comprise types, frequencies and intensities of exercises that work for regulars REG to reach their Peak Performances PP, for use by the irregulars IRR.

Advantageously, the machine learning based model used for the group of regulars REG may be used to predict which individuals of the group of irregulars IRR having similar combination of key physiological parameters KPD and key medical parameters KMP values (i.e. class CLA) may be targeted for the electronic training plan ETP generation.

The computing device 20 sends in a step E9, via the communication network 30, the electronic training plan ETP of each class CLA to the irregulars IRR of said class CLA. Thus, irregulars IRR having similar health and/or physiological parameters than the regular REG are targeted to follow the same electronic training plan ETP.

Advantageously, physiological data reflecting physiological changes may be measured and collected for the irregulars IRR of each class CLA in order to tune their electronic training plan ETP.

Preferably, physiological changes of the irregulars IRR of each class CLA may be monitored and measured over a time period to see how their fitness level is improving. The productivity of the irregulars of each class CLA may also be measured over a period of time.

The method may advantageously be carried out over a period of time called training period.

Once the targeted individuals of the group of irregulars IRR do the prescribed activities on regular basis, e.g. during 3 or 6 months (the training period) or finetune the activities under advice and they start getting peak performances PP and improvements, they can be enrolled into the group of regulars REG. In the opposite, if some individuals of the group of regulars REG do not remain regular, they should be marked as individuals of the group of irregulars IRR, as otherwise they will add errors into the machine learning model prediction.

For improving the efficiency of the method, medical and physiological data of each individual may be aggregated into the database 10 for further recalibration of the Artificial Intelligence-Machine Learning models. For example, key physiological parameters KPD and key medical parameters KMP data available may be gathered over a time period then, this dataset may be split into a training subset and a testing subset. The training dataset is passed through various machine learning training models and the best (i.e. more efficient) machine learning training model is selected. The machine learning training model may be finetuned by applying data engineering techniques to improve the machine learning performance. The trained model is validated on the test dataset and then used to predict the next peak performances PP of the individuals of the group of regulars REG. The trained model is used to generate an electronic training plans ETP for individuals of the group of irregulars IRR having similar key physiological parameters KPD and key medical parameters KMP values (i.e. classes of irregulars). If the individuals of the group of irregulars IRR achieve peak performances PP, they become members of the group of regulars REG for the next machine learning training cycle. If the individuals of the group of regulars REG become irregulars, they become members of the group of irregulars IRR for the next machine learning training cycle.

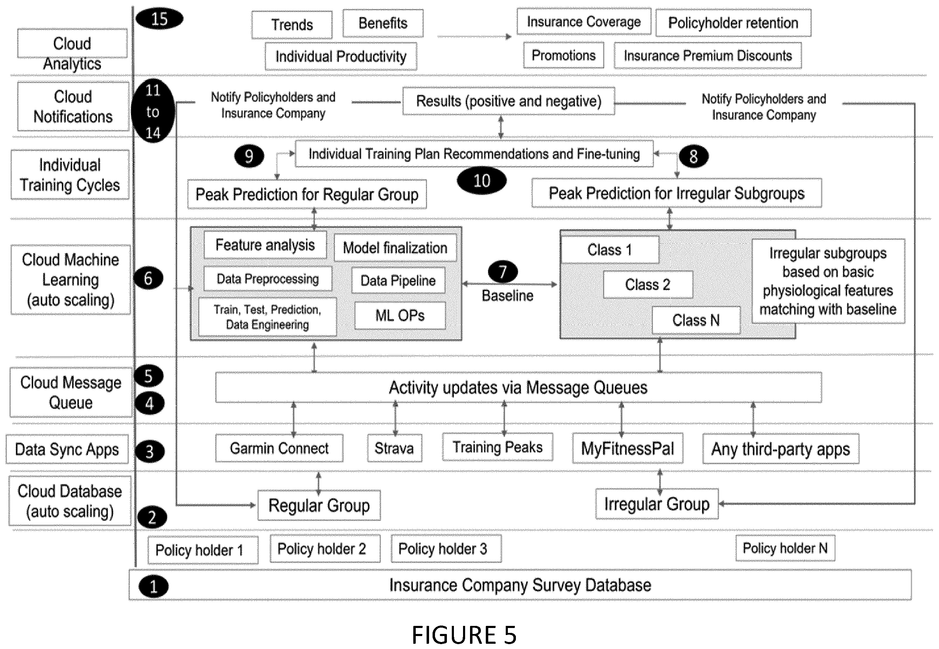

Figure 5 illustrates a detailed example of implementation of the method according to the invention.
1. Using surveys, collect basic health and work lifestyle data at the health insurer level. This can be done before or after issuing a health policy and could be updated on six months basis (for example).
2. Form two member groups: those who are consistent in doing physical activities REG ("regular") and those who are not ("irregular") IRR.
3. Get an individual workout activity related data or using REST APIs integrated with training apps like GarminConnect^{®}, TrainingPeaks^{®} or Strava^{®}, collect the policyholder data related to physiological parameters.
4. For all regular folks, collect the data of their physiological parameters combined with their peak performances in physical activities. A peak performance could be goal specific to that individual e.g. (specific to running activity) time for a 5K race, threshold pace, time spent in a heart rate zone, training volume over a month, long runs etc and correlate the data with work lifestyle parameters.
5. Using hyperscalar cloud platforms (e.g AWS), build databases set for autoscaling and integrated to receive data from IOT devices using message queues and send data using message queues.
6. Using AI-ML, analyse a regular individual's data over a period (say 6 months to a year) to find the key dependent parameters and label the data where the previous peak performance is achieved. Note that a peak performance is very much individual specific. The details of ML model are explained in reference to Figure 2 here above.
7. Categorize the regular members with similar health and/or work lifestyle parameters like age, weight, working hours etc into classes and analyse how they are adapting to stress changes using physical activities, keeping up with and achieving a peak performance to form a baseline.
8. Based on the normalized data of regular member groups, recommend a training plan to irregular individuals having similar health and/or work lifestyle parameters
9. Using AI-ML modeling techniques, find what physical activities could be prescribed and use modelling algorithms to predict the next performance peak. This step could be used to regular group members also.
10. Analyse the physiological changes using the individual's data in irregular group and provide suggestions to tune appropriate training parameters.
11. Monitor and measure the physiological changes over a time period in the irregular group and see how the fitness level for the individual is improving. Also measure how the productivity of that individual has changed over that time period.
12. Once the training cycle is over, notify the policyholder and the insurance company.
13. For positive results, merge the individual's data into the global database so that it could be used for further recalibration of the AI-ML models.
14. For negative results, manually analyze the individual's data to find out what corrections could be made into the training cycle.
15. As the individual's productivity is improved and reported, pass on the benefit to that policyholder in terms of %-reduction in the insurance cost for the next year. Examples could be those who did not claim any hospitalization expenses in the past year, those who had lesser doctor visits than past year etc. The data analytics leading to finding various trends, benefits, improved individual productivity can be used for the purpose of promotions, policyholder's retention, offering premium discounts or better insurance coverage etc.

The individual's productivity may be assessed periodically to monitor improvements and adapt electronic training plans accordingly.

The method according to the invention may be used for example for health insurance. The data of the irregulars IRR rare monitored periodically, e.g. every month and when their health improves continuously through the received electronic training plan ETP over a predetermined period of time, for example six months, their health insurance policy may be reviewed in order to increase their coverage and/or reduce costs for the individual.

The method according to the invention demonstrates a technical solution to strike a work life balance for a working professional using cloud and machine learning models. It presents high level and detailed technical solution, its various components and typical interaction among them to come up with a working model that can be used e.g., by an insurance provider and promote better community health and customer commitment.

## Claims

1. A method for automatically providing an electronic training plan (ETP) to each individual of a targeted group of individuals called "irregulars" (IRR), said targeted group comprising individuals who do not perform at least one activity on a periodic basis, the targeted group being a part of a larger group called set of individuals (SIND), said set of individuals (SIND) comprising another group called "regulars" (REG), disjointed from the group of irregulars (IRR) and comprising individuals who do perform at least one activity on a periodic basis, each individual of the set of individuals (SIND) being **characterized by** a set of medical data (MD) and a set of physiological data (BPD, DPD), said set of physiological data (BPD, DPD) comprising basic physiological data (BPD) and dynamic physiological data (DPD), said basic physiological data (BPD) and said medical data (MD) being stored in a database (10), said method comprising the steps, operated by a computer system (1), of:
- measuring dynamic physiological data (PD) of each individual of the set of individuals (SIND),
- sending said measured dynamic physiological data (DPD) to said database (10) via a communication network (30),
- associating, for each individual, their dynamic physiological data (DPD) with their basic physiological data (BPD) and their medical data (MD),
- collecting a data subset of the set of physiological data (BPD, DPD) and a data subset of the set of medical data (MD) for each individual of the set of individuals, said medical data (MD) subset comprising at least the age and the weight of each individual,
- for each individual of the group of regulars (REG), determining in the collected physiological data subset the data which are correlated with peak performances (PP) and deriving parameters associated with the determined peak performances (PP) data,
- determining a plurality of subsets of the set of individuals (SIND), called "classes" (CLA), based on the medical data (MD) subset and/or the basic physiological data (BPD) of each individual in order to group individuals at least by age and/or weight categories,
- generating, for each class (CLA), a common electronic training plan (ETP) based on the derived parameters of the regulars (REG) of said class (CLA),
- sending to each class (CLA), via the communication network (30), said electronic training plan (ETP) to the irregulars (IRR) of said class (CLA).

2. The method according to claim 1, wherein each individual of the set of individuals (SIND) being associated with a set of basic physiological data (BPD), said method comprises a preliminary step of separating the individuals of the set of individuals (SIND) using the sets of basic physiological data (BPD) of said individuals to define the group of regulars (REG) and the group of irregulars (IRR).

3. The method according to any one of the preceding claims, comprising the step of collecting a set of basic physiological data (BPD) and a set of medical data (MD).

4. The method according to any one of the preceding claims, comprising, subsequently to the generation of a common electronic training plan (ETP) for the irregulars of a class (CLA), a step of tuning said common electronic training plan (ETP) for each irregular of said class (CLA) using the physiological data (PD) subset and/or the medical data subset of said irregular.

5. The method according to any one of the preceding claims, comprising measuring dynamic physiological data (DPD) of each individual of the set of individuals (SIND) using at least one measurement module (5), preferably a plurality of measurement modules (5).

6. The method according to any one of the preceding claims, comprising measuring dynamic physiological data (DPD) of each individual of the set of individuals (SIND) on a periodic basis to update said data regularly, for example at least once a week.

7. The method according to any one of the preceding claims, comprising monitoring specifically dynamic physiological data (DPD) of the individuals of the group of irregulars (IRR) and suggesting improved electronic training plan (ETP) based on the monitored dynamic physiological data (DPD).

8. A computer program comprising instructions which, when the program is executed by a computer, cause the computer to carry out the steps of the method according to any one of the preceding claims.

9. A computing device (20) for automatically providing an electronic training plan (ETP) to each individual of a targeted group of individuals called "irregulars" (IRR), said targeted group comprising individuals who do not perform at least one activity on a periodic basis, the targeted group being a part of a larger group called set of individuals (SIND), said set of individuals comprising another group called "regulars" (REG), disjointed from the group of irregulars (IRR) and comprising individuals who do perform at least one activity on a periodic basis, each individual of the set of individuals (SIND) being **characterized by** a set of medical data (MD) and a set of physiological data (BPD, DPD), said set of physiological data (BPD, DPD) comprising basic physiological data (BPD) and dynamic physiological data (DPD), said device (20) being configured to be operatively coupled to a database (10) comprising for each individual said set of basic physiological data (BPD) and said of medical data (MD), and to:
- receive, via a communication network (30), a data subset of the set of physiological data (BPD, DPD) and a data subset of the set of medical data (MD) for each individual of the set of individuals (SIND), said medical data (MD) subset comprising at least the age and the weight of each individual,
- for each individual of the group of regulars (REG), determine in the collected physiological data subset the data which are correlated with peak performances (PP) and deriving the parameters associated with the determined peak performances (PP) data,
- determine a plurality of subsets of the set of individuals (SIND), called "classes" (CLA), based on the medical data (MD) subset and/or the basic physiological data (BPD) of each individual in order to group individuals at least by age and/or weight categories,
- generate, for each class (CLA), a common electronic training plan (ETP) based on the derived parameters of the regulars (REG) of said class (CLA),
- send to each class (CLA), via the communication network (30), said electronic training plan (ETP) to the irregulars (IRR) of said class (CLA).

10. The device (20) according to the preceding claim, wherein each individual of the set of individuals (SIND) being associated with a set of basic physiological data (BPD), said device (20) being configured to retrieve at least basic physiological data (BPD) for each individual of the set of individuals (SIND) from the database (10) and to separate the individuals of the set of individuals (SIND) using said sets of basic physiological data (BPD) of said individuals to define the group of regulars (REG) and the group of irregulars (IRR).

11. The device (20) according to any one of claims 9 or 10, further configured to collect a set of basic physiological data (BPD) from each individual of the set of individuals (SIND) and store said set in the database (10).

12. The device (20) according to any one of claims 9 to 11, configured, subsequently to the generation of a common electronic training plan (ETP) for the irregulars (IRR) of a class (CLA), to tune said common electronic training plan for each irregular (IRR) of said class (CLA) using the physiological data (PD) subset and/or the medical data (MD) subset of said irregular (IRR).

13. The device (20) according to any one of claims 9 to 12, comprising monitoring specifically the physiological data (PD) of the individuals of the group of irregulars (IRR) and suggesting improved electronic training plan (ETP) based on the monitored dynamic physiological data (PD).

14. The device (20) according to any one of claims 9 to 13, further comprising the database (10).

15. A system (1) for automatically providing an electronic training plan (ETP) to each individual of a targeted group of individuals called "irregulars" (IRR), said targeted group comprising individuals who do not perform at least one activity on a periodic basis, the targeted group being a part of a larger group called set of individuals (SIND), said set of individuals comprising another group called "regulars" (REG), disjointed from the group of irregulars (IRR) and comprising individuals who do perform at least one activity on a periodic basis, each individual of the set of individuals (SIND) being **characterized by** a set of physiological data (PD) and a set of medical data (MD), said system (1) comprising:
- a plurality of measurement modules (5),
- at least one database (10) configured to receive and store medical data (MD) of each individual of the set of individuals (SIND), to receive and store physiological data (PD) of each individual of the set of individuals (SIND) and to associate, for each individual, their physiological data (PD) with their medical data (MD),
- at least one device (20) according to any one of the preceding claims 9 to 14, operatively coupled to said at least one database (10) via at least one communication network (30).
